# EUROPÄISCHE PATENTANMELDUNG

(11) **EP 1 469 042 A2**
(43) Veröffentlichungstag der Anmeldung: **20.10.2004**
(21) Anmeldenummer: 04006077.4
(22) Anmeldetag: 15.03.2004
(51) Int. Cl.: C09C 1/00

(54) **Pigmentgemisch und dessen Verwendung in der Kosmetik und im Lebensmittel- und Pharmabereich**

(30) Priorität: 27.03.2003 DE 10313981; 01.07.2003 DE 10329780
(71) Anmelder: MERCK PATENT GmbH, 64293 Darmstadt (DE)
(72) Erfinder: Hochstein, Veronika, 76646 Bruchsal (DE); Schoen, Sabine, Dr., 45701 Herten (DE)

(57) **Zusammenfassung**

Die vorliegende Erfindung betrifft Pigmentmischungen bestehend aus mindestens zwei Komponenten, wobei Komponente A Effektpigmente auf Basis von Glasplättchen und Komponente B plättchenförmige, nadelförmige, sphärische oder kristalline Farbmittel oder Füllstoffe sind, sowie deren Verwendung insbesondere in kosmetischen Formulierungen und zur Einfärbung oder als Überzug von Produkten im Lebensmittel- und Pharmabereich.

## Beschreibung

Die vorliegende Erfindung betrifft ein Pigmentgemisch bestehend aus mindestens zwei Komponenten, wobei Komponente A Effektpigmente auf Basis von dünnen Glasplättchen und Komponente B plättchenförmige, nadelförmige, sphärische oder kristalline Farbmittel und/oder Füllstoffe sind, sowie dessen Verwendung in kosmetischen Formulierungen und im Lebensmittel- und Pharmabereich.

Beschichtete und unbeschichtete Glasplättchen sind bekannt, z.B. aus der WO 97/46624, WO 03/006558 und WO 02/090448. Deckvermögen und Glanz sind bei plättchenförmigen Pigmenten oftmals nur schwer gleichzeitig in befriedigendem Ausmaß zu realisieren. So zeichnen sich etwa mit einer oder mehreren dünnen Metalloxidschichten belegte Glimmerplättchen oder SiO₂-Plättchen durch Interferenzfarben und einen hohen Glanz, gleichzeitig aber auch aufgrund des durchsichtigen Substrats durch eine hohe Transparenz und damit ein vergleichsweises geringes Deckvermögen, aus.

Effektpigmente auf der Basis von Glasplättchen zeichnen sich durch ihren hohen Glanz, ihre Farbreinheit und Farbstärke aus und sollten daher insbesondere für die Kosmetik interessant sein.

Aufgabe der vorliegenden Erfindung ist es Effektpigmente auf der Basis von Glasplättchen in der Weise bereitzustellen, dass sie ein vergleichsweise hohes Deckvermögen aufweisen, sich gut in das jeweilige Anwendungssystem einarbeiten lassen und gleichzeitig die optischen Eigenschaften, wie insbesondere der Glanz und die Farbreinheit, gar nicht oder nur unwesentlich beeinflusst werden.

Überraschenderweise wurde nun ein Pigmentgemisch gefunden, das keine der oben angegebenen Nachteile aufweist. Das erfindungsgemäße Pigmentgemisch besteht aus mindestens zwei Komponenten, wobei Komponente A Effektpigmente auf Basis dünner Glasplättchen und Komponente B plättchenförmige, nadelförmige, sphärische oder kristalline Farbmittel und/oder Füllstoffe sind.

Durch die Zumischung eines oder mehrerer Farbmittel zu den beschichteten Glasplättchen kann den Anwendungssystemen ein Regenbogeneffekt verliehen werden, der Farbeffekt wird verstärkt und neuartige Farbeffekte werden erzielt. Weiterhin zeichnen sich die Pigmentgemische durch ihren hohen Glanz, einen Glitzereffekt und ihr Hautgefühl aus.

Gegenstand der Erfindung ist somit ein Pigmentgemisch bestehend aus mindestens zwei Komponenten, wobei Komponente A Effektpigmente auf Basis von dünnen Glasplättchen und Komponente B plättchenförmige, nadelförmige, sphärische oder kristalline Farbmittel und/oder Füllstoffe sind.

Gegenstand der Erfindung sind ebenfalls kosmetische Formulierungen, wie z. B. Make-ups, Presspuder, lose Puder, Lippenstifte, Lotions, Emulsionen, etc., die das erfindungsgemäße Pigmentgemisch enthalten. Weiterhin geeignet sind die Pigmentgemische für Einfärbungen und farbige Überzüge von Lebensmitteln und Pharmaprodukten, wie z.B. Arzneimittelüberzüge von Tabletten, Dragees, Gelatinekapseln, etc.

Die beschichteten Glasplättchen können in jedem Verhältnis mit dem Farbmittel bzw. Füllstoff gemischt werden. Vorzugsweise ist das Verhältnis von Komponente A zu Komponente B 95 : 5 bis 5 : 95, insbesondere 80 : 20 bis 20 : 80, ganz besonders bevorzugt 70 : 30 bis 30 : 70.

Bevorzugte Effektpigmente besitzen folgenden Aufbau:
Glasplättchen + TiO₂-Schicht
Glasplättchen + SiO₂-Schicht + TiO₂-Schicht
Glasplättchen + Fe₂O₃-Schicht
Glasplättchen + SiO₂-Schicht + Fe₂O₃-Schicht
Glasplättchen + Fe₃O₄-Schicht
Glasplättchen + SiO₂-Schicht + Fe₃O₄-Schicht
Glasplättchen + TiFe₂O₃-Schicht
Glasplättchen + SiO₂-Schicht + TiFe₂O₃-Schicht
Glasplättchen + Cr₂O₃-Schicht
Glasplättchen + SiO₂-Schicht + Cr₂O₃-Schicht
Glasplättchen + TiO₂-Schicht + Cr₂O₃-Schicht
Glasplättchen + SiO₂-Schicht + TiO₂-Schicht + Cr₂O₃-Schicht
Glasplättchen + Titansuboxid
Glasplättchen + SiO₂-Schicht + Titansuboxid
Glasplättchen + TiO₂-Schicht + Fe₂O₃-Schicht
Glasplättchen + SiO₂-Schicht + TiO₂-Schicht + Fe₂O₃-Schicht
Glasplättchen + TiO₂-Schicht + Berliner Blau
Glasplättchen + SiO₂-Schicht + TiO₂-Schicht + Berliner Blau
Glasplättchen + TiO₂-Schicht + Carminrot
Glasplättchen + SiO₂-Schicht + TiO₂-Schicht + Carminrot
Glasplättchen + TiO₂-Schicht + DC Red 30
Glasplättchen + SiO₂-Schicht + TiO₂-Schicht + DC Red 30
Glasplättchen + Fe₂O₃-Schicht + SiO₂-Schicht + Fe₂O₃-Schicht
Glasplättchen + Fe₂O₃-Schicht + SiO₂-Schicht + TiO₂-Schicht
Glasplättchen + TiO₂-Schicht + SiO₂-Schicht + Fe₂O₃-Schicht
Glasplättchen + TiO₂-Schicht + SiO₂-Schicht + TiO₂/Fe₂O₃-Schicht
Glasplättchen + TiO₂/Fe₂O₃-Schicht + SiO₂-Schicht + TiO₂/Fe₂O₃-Schicht
Glasplättchen + TiO₂-Schicht + SiO₂-Schicht + Cr₂O₃-Schicht

Die TiO₂-Schicht kann in der Modifikation Anatas oder Rutil vorliegen. Besonders bevorzugt sind Glasplättchen, die zunächst mit einer ersten Schicht aus SiO₂ belegt sind und darauf eine oder mehrere Metallschichten besitzen und gegebenenfalls noch mit Karminrot, Berliner Blau oder einem organischen Farbstoff beschichtet sind. Die Metalloxide sind vorzugsweise aus der Gruppe TiO₂, Ti-Suboxide, Fe₂O₃, Fe₃O₄ oder Mischungen davon.

Anstelle der äußeren Metalloxidschicht kann auch eine semitransparente Schicht eines Metalls verwendet werden. Geeignete Metalle dafür sind beispielsweise Cr, Ti, Mo, W, Al, Cu, Ag, Au oder Ni.

Die Dicke der Glasplättchen beträgt vorzugsweise ≤ 1 µm, insbesondere ≤ 0,8 µm und insbesondere bevorzugt ≤ 0,6 µm.

Beschichtete Glasplättchen sind beispielsweise im Handel erhältlich unter der Marke Ronastar , Fa. Merck KGaA.

Zur Erzielung spezieller Farbeffekte können in die hoch- bzw. niedrigbrechenden Schichten zusätzlich noch feinteilige Partikel im Nanometergrößenbereich eingebracht werden. Als geeignet dafür erweisen sich beispielsweise feinteiliges TiO₂ oder feinteiliger Kohlenstoff (Ruß) mit Teilchengrößen im Bereich von 10-250 nm. Durch die lichtstreuenden Eigenschaften derartiger Partikel kann gezielt auf Glanz und Deckvermögen Einfluß genommen werden.

Die Effektpigmente können auch zur Verbesserung der Licht-, Wetter- und chemischen Stabilität oder zur Erhöhung der Kompatibilität in unterschiedliche Medien noch mit einer Schutzschicht versehen sein. Als Nachbeschichtungen bzw. Nachbehandlungen kommen beispielsweise Silane, Silikone, adsorbierenden Silikone, Metallseifen, Aminosäuren, Lecithine, Fluorkomponenten, Polyethylene, Kollagen oder die in den DE 22 15 191, DE 31 51 354, DE 32 35 017 oder DE 33 34 598 beschriebenen Verfahren in Frage. Die zusätzlich aufgebrachten Stoffe machen nur etwa 0,1 bis 5 Gew.%, vorzugsweise 0,5 bis 3,0 Gew.%, des Pigments aus.

Als Komponente B für die erfindungsgemäße Pigmentmischung sind alle dem Fachmann bekannten plättchenförmigen, nadelförmigen, sphärischen und kristallinen Farbmittel oder Füllstoffe geeignet, insbesondere solche, die eine Partikelgröße von 0,001 bis 10 µm, vorzugsweise 0,01 bis 1 µm, aufweisen. Bevorzugt enthalten die erfindungsgemäßen Pigmentgemische als Farbmittel Absorptionspigmente und als Füllstoffe plättchenförmige oder sphärische Pulver. Komponente B sind vorzugsweise beschichtete oder unbeschichtete SiO₂-Kugeln. Mit ein oder mehreren Metalloxiden beschichtete SiO₂-Kugeln sind z.B. aus der EP 0 803 550 A2 bekannt.

Bevorzugte Pigmentgemische enthalten neben Komponente A als Farbmittel (Komponente B) insbesondere ein Perlglanzpigment, einschließlich Mehrschichtpigmente oder Interferenzpigmente. Als Perlglanzpigmente werden Pigmente auf der Basis plättchenförmiger, transparenter oder semitransparenter Substrate aus z.B. Schichtsilikaten, wie etwa natürlichem oder synthetischem Glimmer, Talkum, Sericit, Kaolin oder anderen silikatischen Materialien verwendet, die mit farbigen oder farblosen Metalloxiden wie z.B. TiO₂, Titansuboxide, Titanoxinitride, Fe₂O₃, Fe₃O₄, SnO₂, Cr₂O₃, ZnO, CuO, NiO und anderen Metalloxiden allein oder in Mischung in einer einheitlichen Schicht oder in aufeinanderfolgenden Schichten beschichtet sind.

Perlglanzpigmente sind z.B. aus den deutschen Patenten und Patentanmeldungen 14 67 468, 19 59 998, 20 09 566, 22 14 454, 22 15 191, 22 44 298, 23 13 331, 25 22 572, 31 37 808, 31 37 809, 31 51 343, 31 51 354, 31 51 355, 32 11 602, 32 35 017 und P 38 42 330 bekannt und im Handel erhältlich, z.B. unter den Marken Iriodin® , Timiron® , Xirona® der Firma Merck KGaA, Darmstadt, Deutschland und/oder Rona, USA. Besonders bevorzugte Pigmentpräparationen enthalten TiO₂/Glimmer-, Fe₂O₃/Glimmer- und/oder TiO₂/Fe₂O₃-Glimmerpigmente.

Weiterhin bevorzugt sind beschichtete oder unbeschichtete BiOCI-Pigmente, mit TiO₂ und/oder Fe₂O₃ beschichtete SiO₂- oder Al₂O₃-Plättchen. Die Beschichtung der SiO₂-Plättchen mit ein oder mehreren Metalloxiden kann z.B. erfolgen wie in der WO 93/08237 (naß-chemische Beschichtung) oder DE-OS 196 14 637 (CVD-Verfahren) beschrieben.

Die beispielsweise aus den deutschen Offenlegungsschriften DE 196 18 563, DE 196 18 566, DE 196 18 569, DE 197 07 805, DE 197 07 806, DE 197 46 067 bekannten Mehrschichtpigmente basieren auf einer plättchenförmigen, transparenten, farbigen oder farblosen Matrix, bestehend aus Glimmer (synthetisch oder natürlich), SiO₂-Plättchen, Glasplättchen, Al₂O₃-Plättchen, Polymerplättchen, und besitzen in der Regel eine Dicke zwischen 0,3 und 5 µm, insbesondere zwischen 0,4 und 2,0 µm. Die Ausdehnung in den beiden anderen Dimensionen beträgt üblicherweise zwischen 1 und 250 µm, vorzugsweise zwischen 2 und 100 µm, und insbesondere zwischen 5 und 40 µm. Die Mehrschichtpigmente bestehen aus der Matrix (Substrat) beschichtet mit Metalloxiden (mindestens 2). Die Beschichtung der Substratplättchen Glimmer, SiO₂-Plättchen, Glasplättchen, Al₂O₃-Plättchen mit mehreren Schichten erfolgt so, dass ein Schichtaufbau vorzugsweise bestehend aus alternierenden hoch- und niedrigbrechenden Schichten entsteht. Vorzugsweise enthalten die Mehrschichtpigmente 2, 3, 4, 5, 6 oder 7 Schichten, insbesondere 3, 4 oder 5 Schichten. Geeignete hochbrechende Metalloxide sind beispielsweise Titandioxid, Zirkonoxid, Zinkoxid, Eisenoxide, Eisen-Titan-Oxide (Eisentitanate) und/oder Chromoxid, insbesondere TiO₂ und/oder Fe₂O₃. Als niedrigbrechende Metalloxide kommen SiO₂ und Al₂O₃ zum Einsatz. Es kann hierfür jedoch auch MgF₂ oder ein organisches Polymer (z.B. Acrylat) eingesetzt werden. Die Beschichtung der Substratplättchen kann z.B. erfolgen wie in der WO 93/08237 (nasschemische Beschichtung) oder DE-OS-196 14 637 (CVD-Verfahren) beschrieben.

Bei den Interferenzpigmenten handelt es sich vorzugsweise um Pigmente auf der Basis von Glimmer, Glasplättchen, SiO₂-Plättchen, die mit farbigen oder farblosen Metalloxiden wie z.B. TiO₂, Titansuboxide, Titanoxinitride, Fe₂O₃, Fe₃O₄, SnO₂, Cr₂O₃, ZnO, CuO, NiO und anderen Metalloxiden allein oder in Mischung in einer einheitlichen Schicht oder in aufeinanderfolgenden Schichten beschichtet sind.

Erfindungsgemäße Gemische beinhalten auch Mischungen von Effektpigmenten auf Basis von Glas (=Komponente A) mit Interferenzpigmenten auf Basis von Glas (= Komponente B).

Als plättchenförmige Farbmittel kommen vor allem Perlglanzpigmente insbesondere auf Basis von Glimmer, SiO₂-Plättchen oder Al₂O₃-Plättchen, die nur mit einer Metalloxidschicht umhüllt sind, Metalleffektpigmente (Al-Plättchen, Bronzen), optisch variable Pigmente (OVP's), Flüssigkristallpolymerpigmente (LCP's) oder holographische Pigmente in Frage.

Zu den sphärischen Farbmitteln zählen insbesondere TiO₂, eingefärbtes SiO₂, CaSO₄, Eisenoxide, Chromoxide, Ruß, organische Farbpigmente, wie z.B. Anthrachinon-Pigmente, Chinacridon-Pigmente, Diketopyrrolo-pyrrol-Pigmente, Phthalocyanin-Pigmente, Azopigmente, Isoindolin-Pigmente. Bei den nadelförmigen Pigmenten handelt es sich vorzugsweise um BiOCl, eingefärbte Glasfasern, α-FeOOH, organische Farbpigmente, wie z.B. Azopigmente, β-Phthalocyanin Cl Blue 15,3, Cromophtalgelb 8GN (Ciba-Geigy), Irgalith Blau PD56 (Ciba-Geigy), Azomethinkupferkomplex Cl Yellow 129, Irgazingelb 5GT (Ciba-Geigy).

Die erfindungsgemäße Pigmentmischung ist einfach und leicht zu handhaben. Die Pigmentmischung kann durch einfaches Einrühren in das Anwendungssystem eingearbeitet werden. Ein aufwendiges Mahlen und Dispergieren der Pigmente ist nicht erforderlich.

Die erfindungsgemäße Pigmentmischung kann zur Pigmentierung von Lebensmitteleinfärbungen, zur Veredlung von Lebensmitteln, z. B. Masse-Einfärbung oder als Überzug, in Arzneimittelüberzügen, z.B. bei Dragees und Tabletten, oder in kosmetischen Formulierungen, wie Lippenstifte, Lipgloss, Eyeliner, Lidschatten, Rouge, Sonnenschutz, Prä-Sun- und After-Sun-Präparate, Make-ups, Body Lotions, Badegele, Seifen, Badesalze, Zahnpasta, Haargele, Mascara, Nagellacke, Preßpuder, Shampoos, lose Puder und Gele, etc., verwendet werden. Die Konzentration der Pigmentmischung im zu pigmentierenden Anwendungssystem liegt in der Regel zwischen 0,1 und 70 Gew.%, vorzugsweise zwischen 0,1 und 50 Gew.% und insbesondere zwischen 1,0 und 10 Gew.%, bezogen auf den Gesamtfestkörpergehalt des Systems. Sie ist in der Regel abhängig vom konkreten Anwendungsfall und kann bei losen Pudern bis zu 100 % betragen.

Das erfindungsgemäße Pigmentgemisch kann auch vorteilhaft in der dekorativen und pflegenden Kosmetik eingesetzt werden. Die Einsatzkonzentration und das Mischungsverhältnis von beschichteten Glasplättchen mit Komponente B, insbesondere organischen und anorganischen Farbpigmenten und Farbstoffen, natürlichen oder synthetischen Ursprungs, wie z.B. Chromoxid, Ultramarin, sphärischen SiO₂- oder TiO₂-Pigmenten, sind abhängig vom Anwendungsmedium und dem Effekt, der erzielt werden soll.

Die Mischung aus beschichteten Glasplättchen mit anderen Pigmenten oder Farbstoffen kann in allen Verhältnissen erfolgen, vorzugsweise beträgt das Verhältnis 1 : 10 bis 10 : 1. Die Einsatzkonzentration reicht von 0,01 Gew.% im Shampoo bis zu 70 Gew. % im Preßpuder. Bei einer Mischung von beschichteten Glasplättchen mit sphärischen Füllstoffen, z. B. SiO₂, kann die Konzentration bei 0,01-70 Gew.% in der Formulierung liegen. Die kosmetischen Produkte, wie z.B. Nagellacke, Lippenstifte, Preßpuder, Shampoos, lose Puder und Gele, zeichnen sich durch besonders interessante Glanzeffekte aus. Der Glitzereffekt in Nagellack kann gegenüber herkömmlichen Nagellacken mit Hilfe der erfindungsgemäßen Pigmentgemische deutlich gesteigert werden.

Das erfindungsgemäße Pigmentgemisch kann weiterhin mit handelsüblichen Füllstoffen gemischt werden. Als Füllstoffe sind z.B. zu nennen natürlicher und synthetischer Glimmer, Glasbeads oder Glaspulver, Nylon Powder, reine oder gefüllte Melaminharze, Talcum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, sowie physikalische oder chemische Kombinationen dieser Stoffe.

Bezüglich der Partikelform des Füllstoffes gibt es keine Einschränkungen. Sie kann den Anforderungen gemäß z. B. plättchenförmig, sphärisch, nadelförmig, kristallin oder amorph sein.

Selbstverständlich können die erfindungsgemäßen Pigmente in den Formulierungen auch mit jeder Art von kosmetischen Roh- und Hilfsstoffen kombiniert werden. Dazu gehören u.a. Öle, Fette, Wachse, Filmbildner, Tenside, Antioxidantien, wie z.B. Vitamin C oder Vitamin E, Stabilisatoren, Geruchsverstärker, Silikonöle, Emulgatoren, Lösemittel wie z.B. Ethanol, oder Ethylacetat oder Butylacetat, Konservierungsmittel und allgemein anwendungstechnische Eigenschaften bestimmende Hilfsstoffe, wie z.B. Verdicker und rheologische Zusatzstoffe wie etwa Bentonite, Hektorite, Siliciumdioxide, Ca-Silicate, Gelatinen, hochmolekulare Kohlenhydrate und/oder oberflächenaktive Hilfsmittel, etc.

Die die erfindungsgemäßen Pigmentgemische enthaltenden Formulierungen können dem lipophilen, hydrophilen oder hydrophoben Typ angehören. Bei heterogenen Formulierungen mit diskreten wässrigen und nichtwässrigen Phasen können die erfindungsgemäßen Pigmentgemische in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 5 und 8 liegen.

Den Konzentrationen der erfindungsgemäßen Pigmentgemische in der Formulierung sind keine Grenzen gesetzt. Sie können - je nach Anwendungsfall - zwischen 0,001 (rinse-off-Produkte, z.B. Duschgele) - 100 % (z.B. Glanzeffekt-Artikel für besondere Anwendungen) liegen.

Die erfindungsgemäßen Pigmente können weiterhin auch mit kosmetischen Wirkstoffen kombiniert werden. Geeignete Wirkstoffe sind z.B. Insect Repellents, anorganische UV-Filter, wie z.B. TiO₂, UV A/BC-Schutzfilter (z.B. OMC, B3, MBC), auch in verkapselter Form, Anti-Ageing-Wirkstoffe, Vitamine und deren Derivate (z.B. Vitamin A, C, E, etc.), Selbstbräuner (z.B. DHA, Erytrolyse u.a.) sowie weitere kosmetische Wirkstoffe, wie z.B. Bisabolol, LPO, VTA, Ectoin, Emblica, Allantoin, Bioflavanoide und deren Derivate.

Organische UV-Filter werden in der Regel in einer Menge von 0,5 bis 10 Gewichtsprozent, vorzugsweise 1 - 8 %, anorganische Filter von 0,1 bis 30% in kosmetische Formulierungen eingearbeitet.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Up, Pflegecremes und Sonnenschutzpräparaten eingesetzt werden.

Als Anwendungsform der kosmetischen Formulierungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie z.B. Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Die kosmetischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsionen werden besonders bevorzugt. Emulsionen, W/O-Emulsionen und O/W-Emulsionen sind in üblicher Weise erhältlich.

Weitere Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Feste Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen photochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die Einfärbung der Pharma- und Lebensmittelerzeugnisse erfolgt, indem das Pigmentgemisch bestehend vorzugsweise aus Pigment und Färbemitteln, wie z.B. natürlichen oder naturidentischen Farbstoffen, in den gewünschten Mengenverhältnissen, dem einzufärbenden Erzeugnis in Mengen von 0,005 bis 15 Gew.%, vorzugsweise 0,01 bus 10ß Gew.%, zugegeben wird.

Als zur Einfärbung geeignete Produkte sind insbesondere zu nennen Überzüge auf allen Arten von Lebensmitteln, insbesondere pigmentierte Zucker- und Schellacküberzüge (alkoholische und wässrige), Überzüge mit Ölen und Wachsen, mit Gummi Arabicum und mit Cellulosearten (z.B.

HPMC = Hydroxypropylmethylcellulose), die Einarbeitung bzw. der Auftrag auf Zuckerwaren, Kuchendekorationen, Komprimate, Dragees, Kaugummis, Gummiwaren, Fondanterzeugnisse, Marzipanerzeugnisse, Füllmassen, Kakao- und Fettglasuren, Schokolade und schokoladenhaltigen Produkten, Speiseeis, Cerealien, Snackprodukte, Überzugsmassen, Tortenspiegel, Zuckerstreuseln, Nonpareilles, Gelee- und Gelatinewaren, Bonbons, Lakritze, Zuckerguss, Zuckerwatte, Fett-, Zucker- und Crememassen, Puddings, Desserts, Tortenguss, Kaltschalen, Limonaden und Brausegetränke, Getränke mit stabilisierenden Additiven, wie z. B. Carboxymethylcellulose, gesäuerte und ungesäuerte Milchproduckte, wie z. B. Quark, Joghurt, Käse, Käserinden, Wursthüllen, etc.
Ein weiteres großes Einsatzgebiet liegt im Pharma- und OTC-Bereich zur Einfärbung bzw. als Überzug von Tabletten, Gelatinekapseln, Dragees, Salben, Hustensaft, etc. In Kombination mit üblichen Coatings wie Polymethacrylaten und Cellulosearten, z.B. HPMC, können die Pigmentgemische vielfältig zur Einfärbung eingesetzt werden.

Gegenstand der Erfindung sind somit auch Formulierungen enthaltend das erfindungsgemäße Pigmentgemisch.

Die nachfolgenden Beispiele sollen die Erfindung erläutern, ohne sie jedoch zu begrenzen.

### Beispiele

### Beispiel 1: - Shimmering Foundation

| Phase A | | | |
|---|---|---|---|
| **Rohstoff** | **Bezugsquelle** | **lNCl** | **[%]** |
| Extender W | Merck KGaA/Rona® | Mica, Cl 77891 | 9,00 |
| < 15 µm | | (Titanium Dioxide) | |
| MICRONA® Matte Yellow | Merck KGaA/Rona® | Mica, Cl 77492 (Iron Oxides) | 4,00 |
| < 15 µm | | | |
| MICRONA® Matte Red | Merck KGaA/Rona® | Cl 77491 (Iron Oxides), Mica | 0,40 |
| < 15 µm | | | |
| MICRONA® Matte Black | Merck KGaA/Rona® | Cl 77499 (Iron Oxides), Mica | 0,30 |
| < 15 µm | | | |
| Glas-Plättchen mit TiO₂ (Interferenz Gold) | Merck KGaA/Rona® | Glass, Silica, Cl 77891 (Titanium Dioxide), Tin Oxide | 4,50 |
| 10 - 80 µm | | | |
| RONASPHERE® | Merck KGaA/Rona® | Silica | 5,00 |
| < 10 µm | | | |

| Phase B | | | |
|---|---|---|---|
| **Rohstoff** | **Bezugsquelle** | **INCl** | **[%]** |
| Blanose 7 HF | Aqualon GmbH | Cellulose Gum | 0,20 |
| Veegum | Vanderbilt | Magnesium Aluminium Silicate | 1,00 |
| Texapon K 1296 | Cognis GmbH | Sodium Lauryl Sulfate | 0,60 |
| Triethanolamin reinst | Merck KGaA/Rona® | Triethanolamine | 0,50 |
| Titriplex III | Merck KGaA/Rona® | Disodium EDTA | 0,25 |
| Methyl-4-hydroxybenzoat | Merck KGaA/Rona® | Methylparaben | 0,15 |
| 1,2-Propandiol | Merck KGaA/Rona® | Propylene Glycol | 10,90 |
| Wasser, demineralisiert | | Aqua (Water) | 42,95 |

| Phase C | | | |
|---|---|---|---|
| **Rohstoff** | **Bezugsquelle** | **lNCl** | **[%]** |
| Isopropylmyristat | Cognis GmbH | Isopropyl Myristate | 8,00 |
| Paraffin dünnflüssig | Merck KGaA/Rona® | Paraffinum Liquidum (Mineral Oil) | 3,60 |
| Crodamol SS | Croda GmbH | Cetyl Esters | 2,60 |
| Monomuls 60-35 C | Cognis GmbH | Hydrogenated Palm Glycerides | 1,70 |
| Stearinsäure | Merck KGaA/Rona® | Stearic Acid | 1,50 |
| EUSOLEX® 6300 | Merck KGaA/Rona® | 4-Methylbenzylidene Camphor | 1,30 |
| EUSOLEX® 4360 | Merck KGaA/Rona® | Benzophenone-3 | 0,50 |
| Rona Care^{TM} Tocopherol acetat | Merck KGaA/Rona® | Tocopheryl Acetate | 0,50 |
| Magnesiumstearat | Merck KGaA/Rona® | Magnesium Stearate | 0,10 |
| Propyl-4-hydroxybenzoat | Merck KGaA/Rona® | Propylparaben | 0,05 |

| Phase D | | | |
|---|---|---|---|
| **Rohstoff** | **Bezugsquelle** | **lNCl** | **[%]** |
| Parfümöl 200529 | Fragrance Resources | Parfum | 0,20 |
| Euxyl K 400 | Schülke & Mayr GmbH | Phenoxethanol, Methyldibromo Glutaronitrile | 0,20 |

### Herstellung:

Alle Bestandteile der Phase C bei ca. 75 °C aufschmelzen und rühren, bis alles geschmolzen ist. Das Wasser der Phase B kalt vorlegen, Blanose mit dem Turrax einhomogenisieren, Veegum einstreuen und erneut homogenisieren. Auf 75 °C erwärmen und unter Rühren die übrigen Bestandteile darin lösen. Inhaltsstoffe der Phase A einrühren. Bei 75 °C unter Rühren die Phase C zugeben und 2 min. homogenisieren. Die Masse unter Rühren auf 40 °C abkühlen, Phase D zugeben. Unter Rühren auf Raumtemperatur weiter abkühlen und auf pH 6,0-6,5 einstellen (z.B. mit Citronensäurelösung).

### Beispiel 2: Duschgel

| Phase A | | | |
|---|---|---|---|
| **Rohstoff** | **Bezugsquelle** | **lNCl** | **[%]** |
| Glas-Plättchen mit TiO₂ und Fe₂O₃ (Goldpigment) 20 - 200 µm | Merck KGaA/Rona® | Glass, Silica, Cl 77891 (Titanium Dioxide), Cl 77491 (Iron Oxides) | 0,10 |
| Keltrol T | Kelco | Xanthan Gum | 0,75 |
| Wasser, demineralisiert | | Aqua (Water) | 64,95 |

| Phase B | | | |
|---|---|---|---|
| **Rohstoff** | **Bezugsquelle** | **lNCl** | **[%]** |
| Plantacare 2000 UP | Cognis GmbH | Decyl Glucoside | 20,00 |
| Texapon ASV 50 | Cognis GmbH | Sodium Laureth Sulfate, Sodium Laureth-8 Sulfate, Magnesium Laureth Sulfate, Magnesium Laureth-8 Sulfate, Sodium Oleth Sulfate, Magnesium Oleth Sulfate | 3,60 |
| Bronidox L | Cognis GmbH | Propylene Glycol, 5-Bromo-5-Nitro-1,3-Dioxane | 0,20 |
| Parfümöl Everest 79658 SB | Haarmann & Reimer GmbH | Parfum | 0,05 |
| 1 % FD&C Blue No. 1 in Wasser | BASF AG | Aqua (Water), Cl 42090 (FD&C Blue No. 1) | 0,20 |

| Phase C | | | |
|---|---|---|---|
| **Rohstoff** | **Bezugsquelle** | **lNCl** | **[%]** |
| Citronensäure Monohydrat | Merck KGaA/Rona® | Citric Acid | 0,15 |
| Wasser, demineralisiert | | Aqua (Water) | 10,00 |

### Herstellung:

Für Phase A das Pigment in das Wasser einrühren. Keltrol T unter Rühren langsam einstreuen und rühren bis es gelöst ist. Die Phasen B und C nacheinander hinzufügen und dabei langsam rühren bis alles homogen verteilt ist. pH-Wert auf 6,0 bis 6,4 einstellen.

### Beispiel 3: Eveliner Gel

| Phase A | | | |
|---|---|---|---|
| **Rohstoff** | **Bezugsquelle** | **lNCl** | **[%]** |
| Glas-Plättchen mit TiO₂ (Silberpigment) 20 - 200 µm | Merck KGaA/Rona® | Glass, Silica, Cl 77891 (Titanium Dioxide), Tin Oxide | 5,00 |
| Xirona® Magic Mauve 5 - 50 µm | Merck KGaA/Rona® | Silica, Cl 77891 (Titanium Dioxide), Tin Oxide | 10,00 |
| Mica Black 10 - 60 µm | Merck KGaA/Rona® | Cl 77499 (Iron Oxides), MICA, Cl 77891 (Titanium Dioxide) | 5,00 |
| RONASPHERE® < 10 µm | Merck KGaA/Rona® | Silica | 2,00 |
| Carbopol ETD 2001 | BF Goodrich | Carbomer | 0,40 |
| Citronensäure Monohydrat | Merck KGaA/Rona® | Citric Acid | 0,00 |
| Wasser, demineralisiert | | Aqua (Water) | 60,00 |

| Phase B | | | |
|---|---|---|---|
| **Rohstoff** | **Bezugsquelle** | **lNCl** | **[%]** |
| Glycerin, wasserfrei | Merck KGaA/Rona® | Glycerin | 4,00 |
| Triethanolamin reinst | Merck KGaA/Rona® | Triethanolamine | 0,90 |
| Luviskol VA 64 Powder | BASF AG | PVP/VA Copolymer | 2,00 |
| Germaben II | ISP Global Technologies | Propylene Glycol, Diazolidinyl Urea, Methylparaben, Propylparaben | 1,00 |
| Wasser, demineralisiert | | Aqua (Water) | 9,70 |

### Herstellung:

Perlglanzpigmente und Ronasphere® im Wasser der Phase A dispergieren. Mit einigen Tropfen Citronensäure ansäuern um die Viskosität zu vermindern, Carbopol unter Rühren einstreuen. Nach vollständiger Lösung die vorgelöste Phase B langsam einrühren und pH-Wert auf 7,0 bis 7,5 einstellen.

### Beispiel 4: Lidschatten

| Phase A | | | |
|---|---|---|---|
| **Rohstoff** | **Bezugsquelle** | **lNCl** | **[%]** |
| Xirona® Caribbean Blue 10 - 60 µm | Merck KGaA/Rona® | Silica, Cl 77891 (Titanium Dioxide), Mica, Tin Oxide | 45,00 |
| Glas-Plättchen mit TiO₂ (Silberpigment) 20 - 200 µm | Merck KGaA/Rona® | Glass, Silica, Cl 77891 (Titanium Dioxide), Tin Oxide | 10,00 |
| BIRON® B 50 2 - 35 µm | Merck KGaA/Rona® | Cl 77163 (Bismuth Oxychloride) | 3,00 |
| Colorona® Dark Blue 10 - 60 µm | Merck KGaA/Rona® | MICA, Cl 77891 (Titanium Dioxide), Cl 77510 (Ferric Ferrocyanide) | 10,00 |
| Magnesiumstearat | Merck KGaA/Rona® | Magnesium Stearate | 2,50 |
| Weißer Ton | Merck KGaA/Rona® | Kaolin | 5,00 |
| Hubersorb 600 | J.M. Huber Corp. | Calcium Stearate | 0,50 |
| Talkum | Merck KGaA/Rona® | Talc | 11,00 |

| Phase B | | | |
|---|---|---|---|
| **Rohstoff** | **Bezugsquelle** | **lNCl** | **[%]** |
| Amerchol L 101 | Amerchol | Lanolin Alcohol, Paraffinum Liquidum (Mineral Oil) | 10,70 |
| Super Hartolan | Croda GmbH | Lanolin Alcohol | 1,00 |
| Ewalin 1751 | H. Erhard Wagner GmbH | Petrolatum | 1,00 |
| Propyl-4-hydroxybenzoat | Merck KGaA/Rona® | Propylparaben | 0,10 |
| Parfümöl Elegance # 79228 D MF | Haarmann & Reimer GmbH | Parfum | 0,20 |

### Herstellung:

Bestandteile der Phase A zusammen geben und vormischen. Anschließend die Pudermischung unter Rühren tropfenweise mit der geschmolzenen Phase B versetzen. Die Puder werden bei 40 bis 50 bar gepresst.

### Beispiel 5: Eye Shadow Gel

| Phase A | | | |
|---|---|---|---|
| **Rohstoff** | **Bezugsquelle** | **lNCl** | **[%]** |
| Xirona® Indian Summer 5 - 50 µm | Merck KGaA/Rona® | Silica, Cl 77491 (Iron Oxides) | 15,00 |
| Glas-Plättchen mit Fe₂O₃ 10 - 80 µm | Merck KGaA/Rona® | Glass, Silica, Cl 77491 (Iron Oxides) | 5,00 |
| RONASPHERE® < 10 µm | Merck KGaA/Rona® | Silica | 3,00 |
| Carbopol ETD 2001 | BF Goodrich GmbH | Carbomer | 0,30 |
| Citronensäure Monohydrat | Merck KGaA/Rona® | Citric Acid | 0,00 |
| Wasser, demineralisiert | | Aqua (Water) | 60,00 |

| Phase B | | | |
|---|---|---|---|
| **Rohstoff** | **Bezugsquelle** | **lNCl** | **[%]** |
| Glycerin, wasserfrei | Merck KGaA/Rona® | Glycerin | 2,00 |
| Germaben II | ISP Global Technologies | Propylene Glycol, Diazolidinyl Urea, Methyl paraben, Propylparaben | 0,20 |
| Triethanolamin reinst | Merck KGaA/Rona® | Triethanolamine | 0,70 |
| Wasser, demineralisiert | | Aqua (Water) | 13,80 |

### Herstellung:

Perlglanzpigmente und Ronasphere® im Wasser der Phase A dispergieren.
Mit einigen Tropfen Citronensäure ansäuern um die Viskosität zu vermindern, Carbopol unter Rühren einstreuen. Nach vollständiger Lösung die vorgelöste Phase B langsam einrühren.

### Beispiel 6: Lidschatten

| Phase A | | | |
|---|---|---|---|
| **Rohstoff** | **Bezugsquelle** | **lNCl** | **[%]** |
| Xirona® Caribbean Blue 10 - 60 µm | Merck KGaA/Rona® | Silica, Cl 77891 (Titanium Dioxide), MICA, TIN Oxide | 20,00 |
| Colorona® Dark Blue 10 - 60 µm | Merck KGaA/Rona® | MICA, Cl 77891 (Titanium Dioxide), Cl 77510 (Ferric Ferrocyanide) | 5,00 |
| Glas-Plättchen mit TiO₂ (Silberpigment) 20 - 200 µm | Merck KGaA/Rona® | Glass, Silica, Cl 77891 (Titanium Dioxide), Tin Oxide | 5,00 |
| Talkum | Merck KGaA/Rona® | Talc | 49,50 |
| Kartoffelstärke | Südstärke GmbH | Solanum Tuberosum (Potato Starch) | 7,50 |
| Magnesiumstearat | Merck KGaA/Rona® | Magnesium Stearate | 2,50 |

| Phase B | | | |
|---|---|---|---|
| **Rohstoff** | **Bezugsquelle** | **INCl** | **[%]** |
| Isopropylstearat | Cognis GmbH | Isopropyl Stearate | 9,14 |
| Cetylpalmitat | Merck KGaA/Rona® | Cetyl Palmitate | 0,53 |
| Ewalin 1751 | H. Erhard Wagner GmbH | Petrolatum | 0,53 |
| Parfümöl Elegance # 79228 D MF | Haarmann & Reimer GmbH | Parfum | 0,20 |
| Propyl-4-hydroxybenzoat | Merck KGaA/Rona® | Propylparaben | 0,10 |

### Herstellung:

Bestandteile der Phase A zusammen geben und vormischen. Anschließend die Pudermischung unter Rühren tropfenweise mit der geschmolzenen Phase B versetzen. Die Puder werden bei 40 bis 50 bar gepresst.

### Beispiel 7: - Nagellack

| **Rohstoff** | **Bezugsquelle** | **lNCl** | **[%]** |
|---|---|---|---|
| Glas-Plättchen mit TiO₂ (Silberpigment) 20 - 200 µm | Merck KGaA/Rona® | Glass, Silica, Cl 77891 (Titanium Dioxide), Tin Oxide | 1,50 |
| Colorona® Oriental Beige 10 - 60 µm | Merck KGaA/Rona® | Mica, Cl 77891 (Titanium Dioxide), Cl 77491 (Iron Oxides) | 0,30 |
| Thixotrope Nagellack-Base 1348 | International Lacquers S.A. | Toluene, Ethyl Acetate, Butyl Acetate, Nitrocellulose, Tosylamide/Formaldehyde Resin, Dibutyl Phthalate, Isopropyl Alcohol, Stearalkonium Hectorite, Camphor, Acrylates Copolymer, Benzophenone-1 | 97,90 |
| Red HO 59 | International Lacquers S.A. | Ethyl Acetate, Butyl Acetate, Nitrocellulose, Phthalic Anhydride/Trimellitic Anhydride/Glycols Copolymer, Cl 15850 (D&C Red No.6), Acetyl Tributyl Citrate, Isopropyl Alcohol, Acrylates Crosspolymer | 0,30 |

### Herstellung:

Das Pigment wird zusammen mit der Lackbase und der Farbdispersion eingewogen, gut mit einem Spatel von Hand vermischt und anschließend 10 min bei 1000 Upm gerührt.

### Beispiel 8: Lip Lacquer

| Phase A | | | |
|---|---|---|---|
| **Rohstoff** | **Bezugsquelle** | **lNCl** | **[%]** |
| Glas-Plättchen mit TiO₂ (Silberpigment) 20 - 200 µm | Merck KGaA/Rona® | Glass, Silica, Cl 77891 (Titanium Dioxide), Tin Oxide | 2,50 |
| Timiron® Splendid Violet 10 - 60 µm | Merck KGaA/Rona® | Cl 77891 (Titanium Dioxide), Mica, Silica | 5,00 |
| Xirona® Indian Summer 5 - 50 µm | Merck KGaA/Rona® | Silica, Cl 77491 (Iron Oxides) | 2,50 |
| Rubis Covapate W 4765 | Les Colorants Wackherr | Ricinus Communis (Castor Oil), Cl 15850 (D&C Red No. 7 Calcium Lake) | 5,00 |

| Phase B: | | | |
|---|---|---|---|
| **Rohstoff** | **Bezugsquelle** | **lNCl** | **[%]** |
| Foralyn 5020-Ft | Hercules BV | Methyl Hydrogenated Rosinate | 20,00 |
| Adeps Lanae | Henry Lamotte GmbH | Lanolin | 18,00 |
| Castor Oil | Henry Lamotte GmbH | Ricinus Communis (Castor Oil) | 13,75 |
| Foral 85-E | Hercules BV | Glyceryl Hydrogenated Rosinate | 12,00 |
| Jojoba Oil | Gustav Heess GmbH | Buxus chinensis (Jojoba Oil) | 5,00 |
| EUSOLEX® 2292 | Merck KGaA/Rona® | Ethylhexyl Methoxycinnamate, BHT | 3,00 |
| Antaron V-216 | ISP Global Technologies | PVP/Hexadecene Copolymer | 4,00 |
| Candelilla Wax 2039 L | Kahl & Co. | Candelilla Cera (Cendalilla Wax) | 3,50 |
| Amerchol L 101 | Amerchol | Lanolin alcohol, Paraffinum Liquidum (Mineral Oil) | 3,00 |
| Rohagit S | Röhm GmbH | Acrylates Copolymer | 1,50 |
| Beeswax white | Merck KGaA/Rona® | Cera Alba (Beeswax) | 1,00 |
| Propyl-4-hydroxybenzoate | Merck KGaA/Rona® | Propylparaben | 0,10 |
| OXYNEX® K liquid | Merck KGaA/Rona® | PEG-8, Tocopherol, Ascorbyl Palmitate, Ascorbic Acid, Citric Acid | 0,05 |

| Phase C | | | |
|---|---|---|---|
| **Rohstoff** | **Bezugsquelle** | **lNCl** | **[%]** |
| Fragrance Tendresse # 75418C | Haarmann & Reimer GmbH | Parfum | 0,10 |

### Herstellung:

Alle Bestandteile der Phase B auf 80 °C erhitzen (ausgenommen Foral 85-E). Zugabe von Foral 85-E unter Rühren. Anschließend Zugabe von Phase A und Phase B zu der geschmolzenen Phase B. Die homogene Schmelze wird in die auf 50 °C vorgewärmten Gießformen gegossen.

### Beispiel 9: Shampoo

| Phase A | | | |
|---|---|---|---|
| **Rohstoff** | **Bezugsquelle** | **lNCl** | **[%]** |
| Glas-Plättchen mit Fe₂O₃ 20 - 200 µm | Merck KGaA/Rona® | Glass, Silica, Cl 77491 (Iron Oxides) | 0,05 |
| Timiron® Splendid Gold 10 - 60 µm | Merck KGaA/Rona® | Cl 77891 (Titanium Dioxide), Mica, Silica | 0.10 |
| Carbopol ETD 2020 | BF Goodrich GmbH | Acrylates/C10-30 Alkyl Acrylate Crosspolymer | 0,90 |
| Wasser, demineralisiert | | Aqua (Water) | 59,80 |

| Phase B: | | | |
|---|---|---|---|
| **Rohstoff** | **Bezugsquelle** | **lNCl** | **[%]** |
| Triethanolamin reinst | Merck KGaA/Rona® | Triethanolamine | 0,90 |
| Wasser, demineralisiert | | Aqua (Water) | 10,00 |

| Phase C: | | | |
|---|---|---|---|
| **Rohstoff** | **Bezugsquelle** | **lNCl** | **[%]** |
| Plantacare 2000 UP | Cognis GmbH | Decyl Glucoside | 20,00 |
| Texapon ASV | Cognis GmbH | Magnesium Oleth Sulfate, Sodium Oleth Sulfate, Magnesium Laureth-8 Sulfate, Sodium Laureth-8 Sulfate, Magnesium Laureth Sulfate, Sodium Laureth Sulfate | 8,00 |
| Bronidox L | Cognis GmbH | Propylene Glycol, 5-Bromo-5-Nitro-1,3-Dioxane | 0,20 |
| Parfümöl Everest 79658 SB | Haarmann & Reimer GmbH | Parfum | 0,05 |

### Herstellung:

Für Phase A das Pigment in das Wasser einrühren. Mit einigen Tropfen Citronensäure (10%ig) ansäuern um die Viskosität zu vermindern und das Carbopol unter Rühren langsam einstreuen. Nach vollständiger Lösung langsam Phase B zugeben. Nacheinander werden nun die Bestandteile der Phase C zugegeben.

### Beispiel 10: Shimmering Body Powder

| Phase A | | | |
|---|---|---|---|
| **Rohstoff** | **Bezugsquelle** | **lNCl** | **[%]** |
| Glas-Plättchen mit TiO₂ und Fe₂O₃ (Gold pigment) 20 - 200 µm | Merck KGaA/Rona® | Glass, Silica, Cl 77891 (Titanium Dioxide), Cl 77491 (Iron Oxides) | 10,00 |

| Phase B: | | | |
|---|---|---|---|
| **Rohstoff** | **Bezugsquelle** | **lNCl** | **[%]** |
| Talc | Merck KGaA/Rona® | Talc | 25,00 |
| Bole white powder | Merck KGaA/Rona® | Kaolin | 29,70 |
| Mica M < 15 µm | Merck KGaA/Rona® | MICA | 15,00 |
| Silk Mica < 50 µm | Merck KGaA/Rona® | MICA | 9,50 |
| RONASPHERE® < 10 µm | Merck KGaA/Rona® | Silica, Cl 77891 (Titanium Dioxide), Cl 77491 (Iron Oxides) | 4,00 |
| MICRONA® Matte Yellow < 15 µm | Merck KGaA/Rona® | MICA, Cl 77492 (Iron Oxides) | 1,00 |
| MICRONA® Matte Red < 15 µm | Merck KGaA/Rona® | MICA, Cl 77491 (Iron Oxides) | 1,00 |
| Propyl-4-hydroxybenzoate | Merck KGaA/Rona® | Propylparaben | 0,30 |

| Phase C | | | |
|---|---|---|---|
| **Rohstoff** | **Bezugsquelle** | **lNCl** | **[%]** |
| Cetiol SQ | Cognis GmbH | Squalane | 2,00 |
| Miglyol 812 N | Sasol Germany GmbH | Caprylic/Capric Triglyceride | 2,00 |
| RonaCare® Tocopherol Acetate | Merck KGaA/Rona® | Tocopheryl Acetate | 0,20 |
| Elegance | Haarmann & Reimer GmbH | Parfum | 0,30 |

### Herstellung:

Alle Bestandteile der Phase B zusammen einwiegen und in einem Mixer homogen mischen. Anschließend Phase C zugeben und weiter mixen, dann Phase A zufügen und kurz vermahlen, bis das Perlglanzpigment gleichmäßig verteilt ist.

### Beispiel 11: Sparkling Body Cream (O/W)

| Phase A | | | |
|---|---|---|---|
| **Rohstoff** | **Bezugsquelle** | **lNCl** | **[%]** |
| Glas-Plättchen mit TiO₂ und Fe₂O₃ (Goldpigment) 10 - 80 µm | Merck KGaA/Rona® | Glass, Silica, Cl 77891 (Titanium Dioxide), Cl 77491 (Iron Oxides) | 1,00 |
| Timiron® Splendid Gold 10 - 60 µm | Merck KGaA/Rona® | Cl 77891 (Titanium Dioxide), Mica, Silica | 3,00 |
| Carbopol ETD 2001 | BF Goodrich GmbH | Carbomer | 0,60 |
| Citronensäure Monohydrat | Merck KGaA/Rona® | Citric Acid | |
| Wasser, demineralisiert | | Aqua (Water) | 39,00 |

| Phase B | | | |
|---|---|---|---|
| **Rohstoff** | **Bezugsquelle** | **lNCl** | **[%]** |
| RonaCare^{TM} Allantoin | Merck KGaA/Rona® | Allantoin | 0,20 |
| 1,2-Propandiol | Merck KGaA/Rona® | Propylene Glycol | 3,00 |
| Euxyl K 400 | Schülke & Mayr GmbH | Phenoxethanol, Methyldibromo Glutaronitrile | 0,10 |
| Chemag 2000 | Chemag AG | Imidazolidinyl Urea | 0,30 |
| Methyl-4-hydroxybenzoat | Merck KGaA/Rona® | Methylparaben | 0,15 |
| Wasser, demineralisiert | | Aqua (Water) | 27,65 |

| Phase C | | | |
|---|---|---|---|
| **Rohstoff** | **Bezugsquelle** | **lNCl** | **[%]** |
| Hostaphat KL 340 N | Clariant GmbH | Dilaureth-4-Phosphate | 3,00 |
| Cetylalkohol | Merck KGaA/Rona® | Cetyl Alcohol | 2,00 |
| Paraffin flüssig | Merck KGaA/Rona® | Paraffinum Liquidum (Mineral Oil) | 10,00 |
| Cetiol V | Cognis GmbH | Decyl Oleate | 6,00 |
| Propyl-4-hydroxybenzoat | Merck KGaA/Rona® | Propylparaben | 0,05 |

| Phase D | | | |
|---|---|---|---|
| **Rohstoff** | **Bezugsquelle** | **lNCl** | **[%]** |
| Triethanolamin | Merck KGaA/Rona® | Triethanolamine | 0,35 |
| Wasser, demineralisiert | | Aqua (Water) | 3,50 |

| Phase E: | | | |
|---|---|---|---|
| **Rohstoff** | **Bezugsquelle** | **INCl** | **[%]** |
| Parfümöl 72979 | Haarmann & Reimer GmbH | Parfum | 0,10 |

### Herstellung:

Das Perlglanzpigment im Wasser der Phase A dispergieren. Eventuell mit einigen Tropfen Citronensäure ansäuern, um die Viskosität zu vermindern. Carbopol unter Rühren einstreuen. Nach vollständiger Lösung die vorgelöste Phase B langsam einrühren. Phase A/B und Phase C auf 80 °C erhitzen, Phase C in Phase A/B einrühren, homogenisieren mit Phase D neutralisieren und unter Rühren abkühlen. Bei 40 °C Parfümöl zugeben, unter Rühren auf Raumtemperatur abkühlen.

### Beispiel 12: Lip Gloss

| Phase A | | | |
|---|---|---|---|
| **Rohstoff** | **Bezugsquelle** | **lNCl** | **[%]** |
| Glas-Plättchen mit TiO₂ (Interferenz Gold) | Merck KGaA/Rona® | Glass, Silica, Cl 77891 (Titanium Dioxide), Tin Oxide | 6,00 |
| Glas-Plättchen mit TiO₂ (Interferenz Blau) | Merck KGaA/Rona® | Glass, Cl 77891 (Titanium Dioxide), Silica, Tin Oxide | 3,00 |

| Phase B | | | |
|---|---|---|---|
| **Rohstoff** | **Bezugsquelle** | **lNCl** | **[%]** |
| Indopol H 100 | BP Amoco | Polybutene | 59,95 |
| Bentone Gel MIO V | Elementis Specialites | Quaternium-18 Hectorite, Propylene Carbonate, Paraffinum Liquidum (Mineral Oil | 20,00 |
| Eutanol G | Cognis GmbH | Octyldodecanol | 6,00 |
| RonaCare^{TM} Tocopherol acetate | Merck KGaA/Rona® | Tocopheryl Acetate | 1,00 |
| Dow Corning 1403 Fluid | Dow Corning | Dimethiconol, Dimethicone | 3,00 |
| Rubis Covapate W 4765 | Les Colorants Wackherr | Ricinus Communis (Castor Oil), Cl 15850 (D&C RED NO. 7 Calcium Lake | 1,00 |
| Propyl-4-hydroxybenzoate | | Propylparaben | 0,05 |

### Herstellung:

Alle Bestandteile der Phase B werden zusammen eingewogen, auf 70° C erhitzt und gut durchgerührt bis eine homogen Masse entstanden ist. Dann werden die Pigmente zugegeben und nochmals durchgerührt. Die homogene Mischung füllt man bei 50 - 60° C ab.

### Beispiel 12: Perlglanzseife

| Phase A | | | |
|---|---|---|---|
| **Rohstoff** | **Bezugsquelle** | **lNCl** | **[%]** |
| Glas-Plättchen mit TiO₂ (Interferenz Grün) 20 - 200 µm | Merck KGaA/Rona® | Glass, Silica, Cl 77891 (Titanium Dioxide), Tin Oxide | 0,50 |

| Phase B: | | | |
|---|---|---|---|
| **Rohstoff** | **Bezugsquelle** | **lNCl** | **[%]** |
| Transparente Seifenbase (pflanzlich) | Dreiring-Seifen seit 1771 | SODIUM PALMATE, AQUA, SODIUM COCOATE, GLYCERIN, PROPYLENE GLYCOL, SORBITOL, SODIUM CHLORIDE, SODIUM HYDROXIDE, TETRASODIUM EDTA, TETRASODIUM ETIDRONATE | 95,00 |
| Wasser, demineralisiert | | AQUA (WATER) | 3,50 |
| Parfümöl Soft Touch 50-40 | Cognis GmbH | PARFUM | 1,00 |

### Herstellung:

Alle Inhaltsstoffe der Phase B werden dreimal mit einem Seifenextruder durch ein 0,2 mm Sieb vermischt und dann ohne Sieb zu Pellets verarbeitet. Anschliessend wird Phase A zugegeben und mit Phase B kurz vermischt. Die Seifenmasse wird erneut in der Seifenstrasse extrudiert, durch eine Sieblochplatte (ca. 2,5mm) verstrangt und abgestanzt.

### Beispiel 13: Perlglanzseife

| Phase A | | | |
|---|---|---|---|
| **Rohstoff** | **Bezugsquelle** | **lNCl** | **[%]** |
| Glas-Plättchen mit TiO₂ (Silberpigment) 20 - 200 µm | Merck KGaA/Rona® | Glass, Silica, Cl 77891 (Titanium Dioxide), Tin Oxide | 0,50 |
| Perfume TS 5925B | Quest International | PARFUM | 1,00 |

| Phase B: | | | |
|---|---|---|---|
| **Rohstoff** | **Bezugsquelle** | **lNCl** | **[%]** |
| Prisavon 1984 | Uniqema | SODIUM PALMATE, SODIUM PALM KERNELATE, AQUA (WATER), GLYCERIN, SORBITOL, PALM ACID, PALM KERNEL ACID, TETRASODIUM EDTA, TETRASODIUM ETIDRONATE | 98,50 |

### Herstellung:

Die Seifenbasis wird gewogen und in einen Amalgamator überführt. Parfüm und Perlglanzpigment werden zusammen in dem selben Behälter eingewogen. Sie werden zusammen vordispergiert um Staubbildung zu vermeiden und um eine einheitliche Beschichtung der Seifennudeln in der Vormischung zu erhalten. Die Mischzeit beträgt ca. 5 Minuten. Die Bestandteile werden dann in den Füllschacht der Mahlmaschine überführt und dreimal dem Veredelungsschritt unterzogen. Anschließend wird die nunmehr homogene Seifenmasse in die Form eines Seifenstückes gebracht. Während dieses Vorganges sollte die Temperatur der Seifenmasse auf 45 °C gebracht werden, um einen maximalen Perlglanzeffekt zu erhalten.

## Patentansprüche

1. Pigmentgemisch bestehend aus mindestens zwei Komponenten, wobei Komponente A Effektpigmente auf Basis von Glas-Plättchen und Komponente B organische und anorganische plättchenförmige, nadelförmige, sphärische oder kristalline Farbmittel und/oder Füllstoffe sind.

2. Pigmentgemisch nach Anspruch 1, **dadurch gekennzeichnet, dass** die Farbmittel der Komponente B Perlglanzpigmente, Mehrschichtpigmente und Interferenzpigmente sind.

3. Pigmentgemisch nach Anspruch 1 oder 2, **dadurch gekennzeichnet, dass** die Effektpigmente der Komponente A folgenden Schichtaufbau besitzen:
Glasplättchen + TiO₂-Schicht
Glasplättchen + SiO₂-Schicht + TiO₂-Schicht
Glasplättchen + Fe₂O₃-Schicht
Glasplättchen + SiO₂-Schicht + Fe₂O₃-Schicht
Glasplättchen + Fe₃O₄-Schicht
Glasplättchen + SiO₂-Schicht + Fe₃O₄-Schicht
Glasplättchen + TiFe₂O₃-Schicht
Glasplättchen + SiO₂-Schicht + TiFe₂O₃-Schicht
Glasplättchen + Cr₂O₃-Schicht
Glasplättchen + SiO₂-Schicht + Cr₂O₃-Schicht
Glasplättchen + TiO₂-Schicht + Cr₂O₃-Schicht
Glasplättchen + SiO₂-Schicht + TiO₂-Schicht + Cr₂O₃-Schicht
Glasplättchen + Titansuboxid
Glasplättchen + SiO₂-Schicht + Titansuboxid
Glasplättchen + TiO₂-Schicht + Fe₂O₃-Schicht
Glasplättchen + SiO₂-Schicht + TiO₂-Schicht + Fe₂O₃-Schicht
Glasplättchen + TiO₂-Schicht + Berliner Blau
Glasplättchen + SiO₂-Schicht + TiO₂-Schicht + Berliner Blau
Glasplättchen + TiO₂-Schicht + Carminrot
Glasplättchen + SiO₂-Schicht + TiO₂-Schicht + Carminrot
Glasplättchen + TiO₂-Schicht + DC Red 30
Glasplättchen + SiO₂-Schicht + TiO₂-Schicht + DC Red 30
Glasplättchen + Fe₂O₃-Schicht + SiO₂-Schicht + Fe₂O₃-Schicht
Glasplättchen + Fe₂O₃-Schicht + SiO₂-Schicht + TiO₂-Schicht
Glasplättchen + TiO₂-Schicht + SiO₂-Schicht + Fe₂O₃-Schicht
Glasplättchen + TiO₂-Schicht + SiO₂-Schicht + TiO₂/Fe₂O₃-Schicht
Glasplättchen + TiO₂/Fe₂O₃-Schicht + SiO₂-Schicht + TiO₂/Fe₂O₃-Schicht
Glasplättchen + TiO₂-Schicht + SiO₂-Schicht + Cr₂O₃-Schicht

4. Pigmentgemisch nach Anspruch 3, **dadurch gekennzeichnet, dass** das Effektpigment der Komponente A auf einem Glasplättchen mit einer Schichtdicke ≤ 1 µm basiert.

5. Pigmentgemisch nach einem der Ansprüche 1 bis 4 **dadurch gekennzeichnet, dass** das Pigmentgemisch zusätzlich die in der Kosmetik üblichen Additive enthält.

6. Pigmentgemisch nach einem der Ansprüche 1 bis 5, **dadurch gekennzeichnet, daß** Komponente A und Komponente B im Verhältnis 95:5 bis 5:95 gemischt sind.

7. Verwendung des Pigmentgemisches nach Anspruch 1 in kosmetischen Formulierungen, zur Veredelung von Lebensmitteln und im Pharmabereich.

8. Formulierungen enthaltend ein Pigmentgemisch nach Anspruch 1.
